# EUROPEAN PATENT APPLICATION

(11) **EP 2 138 840 A1**
(43) Date of publication of application: **30.12.2009**
(21) Application number: 08382023.3
(22) Date of filing: 27.06.2008
(51) Int. Cl.: G01N 33/15, G01N 13/02, G01N 21/64, G01N 21/77

(54) **A method for evaluating the surface activity of test preparations**

(71) Applicant: Universidad Complutense De Madrid, 28040 Madrid (ES)
(72) Inventor: Ravasio, Andrea, 6094, AXAMS (AT); Cruz, Antonio, 28017 MADRID (ES); Pérez Gil, Jesús, 28040 MADRID (ES); Haller, Thomas, 6094 GRINZENS (AT); Echaide Torreguitar, Mercedes, Universidad Complutense de Madrid 28040 MADRID (ES)
(74) Representative: Barlocci, Anna

(57) **Abstract**

The present invention relates to a method and a kit for evaluating the surface activity of a test preparation comprising: incubating a labelled test preparation with a non-surface active extinguishing agent which blocks the detectable signal of the labelled preparation; detecting the signal produced by the labelled preparation adsorbed into interfacial air-liquid interface; and comparing the signal obtained with the signal produced by a standard surfactant. It also relates to a method for determining the influence of experimental parameters, and the influence of inhibitor/activators on the surface activity; to a method for screening a substance library, and to a method for controlling the quality of a surfactant.

## Description

The present invention relates to a method and a kit for evaluating the surface activity of a test preparation. It also relates to a method for determining the influence of experimental parameters, and inhibitor/activators on the surface activity of a surfactant preparation; to a method for screening a substance library, and to a method for controlling the quality of a surfactant.

### BACKGROUND ART

Lung surfactant is a complex mixture of phospholipids and proteins, which acts as a surface active material, able to reduce the surface tension at the respiratory air-liquid interface of lungs, thus preventing collapse of the alveoli at end expiration. Functional lung surfactant quickly adsorbs into the interface from the first air breath and efficiently replaces surfactant "spent" during subsequent respiratory cycling.

Beside constitutive disorders or immaturity of the lungs at birth, many environmental factors and pathological events can perilously impair the surfactant system and, consequently, lead to pulmonary dysfunctions.

Most clinical surfactants currently in use, however, consist of relatively crude extracts from animal sources and several attempts have been made towards the development of new entirely synthetic human-like therapeutic surfactant preparations. On the other hand, clinical surfactants available today have proven not to be effective enough to ameliorate patients with Acute Respiratory Distress Syndrome, the major cause of mortality in critical care units.

A good surfactant must show very rapid interfacial adsorption to form surface films with equilibrium surface pressures around 45-48 mN/m (24-27 mN/m surface tension) in few seconds, but also must be able to reduce surface tension to values in the order of 1-2 mN/m (70-72 mN/m surface pressure) upon repetitive compression-expansion cycling. Such surface properties can be evaluated in traditional surface balances, in the so-called pulsating bubble surfactometer (PBS) (G. Enhorning "Pulmonary surfactant function studied with the pulsating bubble surfactometer (PBS) and the capillary surfactometer (CS)" Comp Biochem Physiol A Mol Integr Physiol 2001, vol. 129, pp. 221-6 and S. L. Seurynck, et al. "Optical monitoring of bubble size and shape in a pulsating bubble surfactometer", J Appl Physiol 2005, vol 99, pp. 624-33) or in more sophisticated captive bubble surfactometer (CBS) (S. Schurch, et al. "Surface activity in situ, in vivo, and in the captive bubble surfactometer", Comp Biochem Physiol A Mol Integr Physiol 2001, vol. 129, pp. 195-207).

Surfactant adsorption as measured in miniaturized King-Clements devices (L. Camacho, et al. "Effect of pH on the interfacial adsorption activity of pulmonary surfactant", Colloids Surf. B: Bioint. 1996, vol. 5, pp. 271-277 and J. Perez-Gil, et al. "Interfacial adsorption of simple lipid mixtures combined with hydrophobic surfactant protein from pig lung", Biochem Cell Biol 1992, vol. 70, pp. 332-8) or the compression isotherms of surfactant as obtained in Langmuir balances (K. Nag, et al. "Phase transitions in films of lung surfactant at the air-water interface" Biophys J 1998, vol. 74, pp. 2983-95) provide detailed measurements that can be correlated with potential configurations and interactions of lipid and protein molecules as organized at the air-liquid interface. The conditions used in these experiments are usually far, however, of relevant environmental constraints such as physiological temperature, relatively large surfactant concentrations and rapid surface cycling. Surfactant can be assessed in a PBS under more physiological-like conditions, being this technique probably the most used in clinical research. However, it has been shown that PBS has problems to produce accurate data on the surface behavior of surfactants at very low tensions (high surface pressures), the part of the compression-expansion isotherms where the difference between optimal and suboptimal surfactant preparations may be critical. Assessment of surfactant preparations in the CBS has produced the most reliable data under physiologically relevant conditions, but requires a substantial effort in terms of data processing. All these techniques have to be applied in a regime of sample-by-sample analysis, which precludes an extensive evaluation of the multiple conditions and parameters thought to govern surfactant function.

Characterization of pulmonary surfactant action in clinically relevant contexts has progressed through the development of clever experimental setups such as the pulsating bubble surfactometer, the capillary surfactometer, or the captive bubble surfactometer, able to offer more physiological-like conditions to surfactant performance tests. The use of the CBS is becoming in this sense the preferred tool of pulmonary surfactant biophysicists as it is the only technique able to produce accurate enough data under the most physiological-like conditions.

At present, screening for substances with potentially harmful effects on surfactant action is a labor intense, costly and technically demanding procedure. Both the detailed evaluation of pathological factors affecting pulmonary surfactant function and the development of new improved clinical surfactants are critically dependent on the availability of reliable methods to qualitatively and quantitatively estimate surfactant activity. Therefore, there is an interest in the development of new methods for evaluating the surfactant adsorption into interfacial air-liquid interface and formation of efficient surface active films.

### SUMMARY OF THE INVENTION

Inventors have developed a new method for evaluating surface activity of surfactant preparations. In contrast to methods based on pure surface tension measurements, this method gives a direct read out of the amount of surfactant reaching the interface and stably associating with the interfacial film. The method not only is useful for evaluating the surface activity of a pulmonary surfactant but also the surfactant activity of any surfactant preparation, for instance, a detergent.

The principle of measurement is simple, robust and highly reproducible. Unlike the known techniques which have to be applied in a regime of sample-by-sample analysis, the new method can be used to implement high throughput screening of substances under a variety of conditions and parameter thought to govern surfactant function, since only a low amount of substance is required in order to be properly assessed.

Therefore, a first aspect of the present invention refers to a method for evaluating the surface activity of a test preparation to form a film at the air-liquid interface comprising the following steps:
a) incubating the test preparation with a detectable substance to obtain a labelled preparation which provides a detectable signal,
b) preparing an aqueous solution comprising a non-surface active extinguishing agent which is able to block the detectable signal of the substance in step a),
c) combining the labelled preparation of step a) with the solution of step b), and mixing the obtained solution for a period of time sufficient to allow the labelled preparation to adsorb into the air-liquid interface and form a surface film,
d) detecting the signal produced by the labelled preparation adsorbed into interfacial air-liquid interface, and
e) comparing the signal obtained in step d) with the signal produced by a standard surfactant.

Another aspect of the present invention relates to a kit for evaluating the surface activity of a test preparation by the method as defined above comprising:
a) a detectable substance with molecular properties making it suitable for stable incorporation into test preparations,
b) a solution comprising a non-surface active extinguishing agent which is able to block the detectable signal of the substance in step a), and
c) a standard surfactant.

The method of the invention offers complementary data to the parameters obtained when surface activity is measured in classical surface balances. Thus, another aspect of the invention relates to a method for evaluating the surface activity of a test preparation comprising carrying out the method as defined above and at least a method for measuring the surface tension.

The surface activity of a surfactant preparation may be altered by several experimental parameters. Therefore, another aspect of the present invention is a method for determining the influence of an experimental parameter on the surface activity of a surfactant preparation, comprising evaluating the surface activity according to the method as defined above in response to variations of the experimental parameter.

Further, the surface activity of a surfactant preparation may be impaired by the presence of a surfactant inhibitor. According to another aspect of the invention, it is provided a method for determining the influence of a surfactant inhibitor on the surface activity of a surfactant preparation, the method comprising evaluating the surface activity according to the method as defined above, further comprising the step of adding the inhibitor, either to the labelled preparation of step a), or alternatively, to the aqueous solution of step b), and in step e) comparing the signal obtained in step d) with the signal produced either by the standard surfactant, or by the surfactant preparation in the absence of inhibitor.

On the other hand, surfactant activators may increase the surface activity of a surfactant preparation, and/or may, in the presence of inhibitors, at least partially counteract the effects of the latter. Thus, according to another aspect of the invention, it is provided a method for determining the influence of a surfactant activator on the surface activity of a surfactant preparation, the method comprising evaluating the surface activity according to the method as defined above, further comprising the step of adding the activator either to the labelled preparation of step a), or alternatively, to the aqueous solution of step b), in the absence or presence of an inhibitor, and in step e) comparing the signal obtained in step d) with the signal produced either by the standard surfactant, or by the surfactant preparation in the absence of activator.

Another aspect of the present invention refers to a method for screening a compound library to determine the ability of the compounds to inhibit or activate the surface activity of a surfactant preparation, the method comprising evaluating the surface activity according to the method as defined above, further comprising the step of adding each compound to be screened either to the labelled preparation of step a), or alternatively, to the aqueous solution of step b).

Finally, another aspect of the invention relates to a method for controlling the quality of a surfactant preparation, the method comprising evaluating the surface activity according to the method as defined above.

These aspects of the present invention will be further described in the detailed description section that follows. Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional aspects, advantages and features of the invention will become apparent to those skilled in the art upon examination of the description or may be learned by practice of the invention.

### BRIEF DESCRIPTION OF THE DRAWINGS

FIG. 1 shows the ability of BB to absorb and extinguish the fluorescence from the water soluble dye LTG.
FIG. 2 shows the surface adsorption of DPPC, a DPPC:POPG mixture and NS as a function of time (FIG. 2 A to 2 C), and as a function of the surfactant amount (FIG. 2 D to 2 F).
FIG. 3 shows the surface adsorption of Lamellar Body-like Particles (LBPs) and treated LBPs, and comparison with a DPPC:POPG mixture and NS.
FIG. 4 shows Wilhelmy Balance evaluation of surface pressure increase due to surfactant adsorption of DPPC, a DPPC:POPG mixture and NS in the absence/presence of BB (FIG. 4 A and B), and direct comparison with fluorescence measurements (FIG. 4 C).
FIG. 5 shows the influence of ionic strength on the surface activity of NS.
FIG. 6 shows the influence of calcium on the surface activity of NS.
FIG. 7 shows the influence of pH on the surface activity of NS when the surfactant is pre-incubated at different pH (FIG. 7 A), and when surfactant is diluted at pH 7 and injected to subphases adjusted to different pH (FIG. 7 B).
FIG. 8 shows the influence of the serum proteins on the surface activity of NS (FIG. 8 A) and of Curosurf® (FIG. 8 B).
FIG.9 shows the influence of the surfactant inhibitor BSA on the surface activity of NS.

### DETAILED DESCRIPTION OF THE INVENTION

As mentioned above, an aspect of the invention is the method for evaluating the surface activity of a test preparation as described above. The term surface activity refers herein to the ability of a surfactant preparation to adsorb into the air-liquid interface and form a surface-associated film.

The surfactants of the present invention comprise molecules with an amphipatic structure having a hydrophobic and a hydrophilic portion, and show affinity for adsorbing into polar/non-polar interfaces, such as the air-liquid interface of an aqueous surface. As a consequence of the distribution of the surfactant molecules in the interface by minimizing the number of water molecules exposed to the air, the surfactant reduces the surface tension, i.e. it shows surface activity.

In a preferred embodiment, the method of the present invention is used to evaluate the surface activity of a test preparation, which is suspected to be useful as pulmonary surfactant. The term pulmonary surfactant is non-limiting, and includes any pulmonary surfactant of natural origin as well as any synthetic pulmonary surfactant-mimicking preparation. Examples of known pulmonary surfactants are phospholipids such as 1,2-dipalmitoyl-sn-glycero-3-phosphocholine (DPPC), 1-palmitoyl-2-oleoyl-sn-glycero-3-phosphocholine (POPC), 1-palmitoyl-2-oleoyl-sn-glycero-3-[phospho-rac-(1-glycerol) (POPG), and mixtures thereof; native surfactant purified from animal bronchoalveolar lavages and lamellar Body-like Particles (LBPs) obtained from isolated pneumocytes (as described in T. Haller et al. "Tracing surfactant transformation from cellular release to insertion into an air-liquid interface", Am J Physiol Lung Cell Mol Physiol 2004, vol.286, pp. L1009-L1015). An example of a clinical surfactant is Curosurf® (INN: Poractant alfa). The surface activity of these surfactants has been tested to prove the efficiency of the method of the present invention.

It has been proposed that a good pulmonary surfactant not only promotes formation of a simple surface layer of surface active phospholipids, but an interfacial film composed of several layers, including probably the monolayer directly exposed to air, responsible for the reduction in tension, and one or several associated surfactant bilayers. The traditional methods based on surface tension measurements strictly inform about the air-exposed side of the film, that is, about the formation of a mere surface active monolayer, which is the only responsible for the decrease in surface tension. In contrast, the method of the invention evaluates the formation of the whole film, which may be of great importance when looking for surfactant structure-function correlations. Thus, the method of the invention offers complementary data to the parameters obtained when examining pulmonary surfactant activity in classical surface balances.

In a first step, the method of the invention comprises incubating the test preparation with a detectable substance. The test preparation, which is suspected to have surfactant properties, must be capable of interact with the detectable substance, so as to provide a stable interaction which results in the formation of a labelled preparation.

The detectable substance is a molecule, being or comprising a detectable moiety, and this molecule interacts stably with the test preparations. The detectable substance can be also a reactive compound suitable to introduce a signal by strong and stable association to any of the components of the test preparation. The detectable substance does not adsorb to the air-liquid interface *per se* nor affect the surface properties of the test preparations upon incorporation into them. The signal generated by this detectable substance should be stable and not much affected by environmental factors.

In a particular embodiment, the detectable moiety is selected from the group consisting of a fluorophore, a luminiscent functional group, and a phosphorescent functional group. In a preferred embodiment, the detectable substance comprises a fluorophore. Non-limiting examples of detectable substances are 1,2-bis-(4,4-difluoro-5,7-dimethyl-4-bora-3a,4a-diaza-s-inda-cene-3-undecanoyl)-sn-glycero-3-phosphocholine (Bodipy-PC) and other Bodipy-labelled phospholipid derivatives, 1,1'-dioctadecyl-3,3,3',3'-tetramethylindocarbocyanine perchlorate (Dialkylcarbocyanine probes as DilC₁₈), N-(7-nitrobenz-2-oxa-1,3-diazol-4-yl)-1,2-dihexadecanoyl-sn-glycero-3-phosphoethanolamine (NBD-PE), 2-(12-(7-nitrobenz-2-oxa-1,3-diazol-4-yl)amino)dodecanoyl-1-hexadecanoyl-sn-glycero-3-phosphocholine (NBD-PC), N-(fluorescein-5-thiocarbamoyl)-1,2-dihexadecanoyl-sn-glycero-3-phos-phoethanolamine (Fluorescein-PE), 2-(3-diethylamino-6-diethylazaniumyli-denexanthen-9-yl)-5-[2-[2,3-di(hexadecanoyloxy)propoxy-hydroxyphosphoryl]oxyethylsulfamoyl]benzenesulfonate]-phosphatidylethanolamine (Rhodamine-PE), and N-(5-( Chlorosulfonyl)- 2-( 1 H, 2H, 3H, 5H, 6H, 7H, 11H, 12H, 13H, 15H, 16H, 17H- pyrido[ 3, 2, 1- ij] quinolizino[ 1', 9': 6, 7, 8] chromeno[ 2, 3- f] quinolin-4-ium-9-yl) benzenesulfonate)1,2-dihexadecanoyl-sn-glycero-3-phosphoethanolamine (Texas Red®-PE). In a more preferred embodiment, the detectable fluorophore is selected from the group consisting of 4,4-difluoro-4-bora-3a,4a-diaza-s-indacene (bodipy) and the alexa type dyes. The latter fluorophores are particularly stable and independent from the environment. In the most preferred embodiment, the fluorophore is bodipy.

In another particular embodiment, the detectable substance only produces a detectable signal when interacts with the test preparation, not producing any signal when this detectable substance is in solution. Non-limiting examples of these substances are *N*-(3-triethyl ammonium propyl)-4-(4-(dibutylamino)styryl) pyridinium dibromide (FM®1-43) and it's analogs (e.g. FM 1-43FX, FM 5-95, FM 4-64, FM 4-64FX). Another example substance is 7-diethylamino-3,4-benzophenoxazine-2-one (Nile Red), which is fluorescent when it is free in an aqueous solution but whose fluorescent quantum yield increases dramatically upon insertion into surfactants.

When the detectable substance is incubated with the test preparation yielding the labelled preparation, a stable interaction between the detectable substance and the test preparation is formed.

In the case of pulmonary surfactants, the test preparations are lipoproteic complexes comprising various components. When the detectable substance is incubated with this type of surfactant, the hydrophobic portion of the detectable substance is inserted into the phospholipid membranes of the lipoproteic complex, and it establishes hydrophobic interactions with the acyl chains of the phospholipids; whereas the hydrophilic portion of the detectable substance establishes polar interactions (hydrogen bonds, dipolar interactions, electrostatic interactions) with the polar head groups of the membrane phospholipids. The affinity of the detectable substance for the membranes of these lipoproteic complexes is determined by the extension of the hydrophobic part of the detectable substance. Thus, the detectable substance must be sufficiently hydrophobic so as to be practically insoluble in water, so that they remain inserted into the membranes of the lipoproteic complexes.

The detectable substance is dissolved in a suitable solvent preferably in a higher concentration as compared to compound to be labelled. The detectable substance is incubated with the test preparation to be labelled at a suitable temperature and for a suitable time. Preferably the labelling is carried out either at 25°C or at 37 °C. After labelling, the detectable substance is removed, for instance by centrifugation. Thus, the measured signal of an aliquot of the labelled preparation may be correlated with the amount of the test preparation.

When the detectable substance is a fluorophore, the incorporation of the detectable substance into the test preparation may be detected by absorption or emission spectroscopy or any other form of photon counting. Thus, the measured intensity of absorption or emission of an aliquot of the labelled preparation may be correlated with the amount of surfactant.

In a second step of the method, an aqueous solution comprising a non-surface active extinguishing agent able to block the signal of the detectable substance is prepared. In a preferred embodiment, the test preparation to evaluate is a pulmonary surfactant and the aqueous solution is a buffered solution. Preferably, the buffered solution shows pH and osmolarity values such as to meet physiological conditions (pH 7.4; 309 mOsm). Non-limiting examples of buffering agents are 2-amino-2-hydroxymethyl-propane-1 ,3-diol (Tris) and 4-(2-hydroxyethyl)-1-piperazine ethanesulfonic acid (HEPES). In a particular embodiment, the aqueous solution comprises one or more salts selected form the group consisting of NaCl, KCI, MgCl₂ and CaCl₂.

As it will be obvious to a person skilled in the art, the steps a) and b) of the method of the invention may be carried out in any order.

The extinguishing agent of the present invention has the ability to block any signal produced by the detectable substance. The concentration of the extinguishing agent which completely blocks the signal of the detectable substance may be determined by a previous dose-response analysis.

As mentioned above, the extinguishing agent must be non-surface active, so that it does not compete with the test preparation for adsorbing into the air-liquid interface. In addition, the extinguishing agent must not interact with any other elements of the method, and must not change substantially the physical properties of the aqueous solution (for instance, increasing the density of the aqueous solution in such a way that diffusion of the test preparations towards the air-liquid interface would be impaired).

In a particular embodiment, the extinguishing agent is selected from the group consisting of a luminiscence extinguisher, a fluorescence extinguisher, and a phosphorescence extinguisher. In a preferred embodiment, the extinguishing agent is a fluorescence extinguisher. Non-limiting examples of fluorescence extinguishers are tetrasodium (6Z)-4-acetamido-5-oxo-6-[[7-sulfonato-4-(4-sulfonatophenyl)diazenylnaphthalen-1-yl]hydrazinylidene]naphthalene-1,7-disulfonate(Brilliant Black®), tetrasodium (3Z)-5-amino-3-[[4-[4-[(2Z)-2-(8-amino-1-oxo-3, 6-disulfonatonaphthalen-2-ylidene)hydrazinyl]-3-methylphenyl]-2-methylphenyl]hydrazinylidene]-4-oxonaphthalene-2,7-disulfonate (Trypan Blue), and India Ink. In the most preferred embodiment the fluorescence extinguishing agent is Brilliant Black®. This extinguishing agent exhibits a high molar extinction coefficient (ε) for both the emission and excitation wavelengths used to monitor Bodipy (ε₄₈₅ₙₘ = 11172 and ε₅₄₀ₙₘ = 3577.3 L/mol x cm).

In a particular embodiment, the aqueous solution containing the extinguishing agent is introduced in a measurement instrument able to measure the detectable signal, and any background signal obtained is eliminated. Thus, the signal measured by the subsequent addition of the labelled preparation into this solution corresponds only to the adsorbed labelled preparation.

Then, the labelled preparation is introduced into the aqueous solution containing the extinguishing agent, and the resulting solution is mixed for a period of time sufficient to allow the labelled preparation to adsorb into interfacial air-liquid interface, and form an efficient surface active film. Preferably, the labelled preparation is injected into the solution and the mixing is carried out by means of agitation, preferably by orbital shaking, which may be continuous, or in cycles.

After that, the signal produced by the labelled preparation adsorbed into the air-liquid interface is detected. Measurements are performed in the top reading mode. This means that only the signal strictly coming from surface-adsorbed material is measured, while the signal of the non-adsorbed labelled preparation passing into or returning from the aqueous solution is blocked by the extinguishing agent.

In a particular embodiment, the method is carried out in a multi-well plate and the instrument used is a plate reader. Preferably, the instrument used in the present invention allows the control of the temperature and the orbital shaking of the entire plate in between single measurements during a kinetic cycle.

In a preferred embodiment, the detectable substance of the kit of the invention comprises a fluorophore and the extinguishing agent is a fluorescence extinguisher, that is, a fluorescence excitation light and fluorescence emission light blocking agent.

The method of the invention may be used for both evaluating whether a test preparation shows surfactant properties, or whether a given surfactant works better or worse than another known surfactant. Therefore, the signal measured for a test preparation is compared to the signal produced by a standard surfactant. This comparison can be done in different ways, and the skilled person in the art will determine, in each case, the best way to carry out the comparison.

Surface adsorption of surfactants is a time and dose dependent process, and these features can be followed either by their kinetics at a given compound concentration (variation of measured signal versus time), or by their endpoints reached at saturation (measured signal versus compound concentration). For example, for a given concentration of the labelled preparation, the detectable signal may be measured as a function of time, and be subsequently compared with the corresponding curve of a standard surfactant at the same concentration. Alternatively, the detectable signal may be measured after a specific time period as a function of the concentration of the labelled preparation.

Different types of surfactants may be used as standard surfactants. For example, the standard surfactant may be purified native surfactant of animal origin. Other non-limiting examples of standard surfactants are synthetic surfactants or mixtures of phospholipids and purified surfactant proteins or peptides.

As already mentioned, the method of the invention offers complementary data to the parameters obtained when surface activity is measured in classical surface balances. In a preferred embodiment, the method of the invention is used in combination with a King-Clements device, with the pulsating bubble surfactometer (PBS), or with the captive bubble surfactometer (CBS).

Moreover, the method of the invention also allows determining the influence of an experimental parameter on the surface activity of a test preparation in response to variations of this parameter. Non-limiting examples of such experimental parameters are the ionic strength, pH, temperature, presence of cations, such as Ca, Mg and Mn; presence of metals, such as Zn, Fe and Ni; presence of chelating agents, such as ethylenediaminetetraacetic acid (EDTA); presence of certain amounts of organic solvents, such as dimethylsulfoxide, methanol, ethanol, acetonitrile and acetone; presence of denaturing agents, such as urea or guanidinium chloride; presence of reducing agents, such as dithiotreitol (DTT) or mercaptoethanol; presence of oxidizing agents, and presence of free radicals. Other parameters which may influence the surface activity of a surfactant preparation and which may be assessed by the method of the invention are the particle size of the surfactant preparations, and the storage conditions at different temperatures for different periods of time, or the storage procedure, e.g. lyophylisation. In a particular embodiment, the experimental parameter assessed is selected from ionic strength, pH, and presence of Ca.

Moreover, the method of the invention also allows determining the influence of a surfactant inhibitor on the surface activity of a test preparation. When this assay is carried out at different concentrations of the inhibitor, the influence thereof on the surface activity may be evaluated. This method can be carried out using two different ways.

In a first approach, the method comprises the following steps:
a) incubating the test preparation with a detectable substance to obtain a labelled preparation which provides a detectable signal,
b') preparing an aqueous solution comprising a non-surface active extinguishing agent which is able to block the detectable signal of the substance in step a), and adding the inhibitor,
c) combining the labelled preparation of step a) with the solution of step b'), and mixing the obtained solution for a period of time sufficient to allow the labelled preparation to adsorb into the air-liquid interface and form a surface film,
d) detecting the signal produced by the labelled preparation adsorbed into interfacial air-liquid interface, and
e) comparing the signal obtained in step d) with the signal produced either by the standard surfactant, or by the surfactant preparation in the absence of inhibitor.

Alternatively, the method comprises the following steps:
a') incubating the test preparation with a detectable substance to obtain a labelled preparation which provides a detectable signal, and adding the inhibitor,
b) preparing an aqueous solution comprising a non-surface active extinguishing agent which is able to block the detectable signal of the substance in step a'),
c) combining the substances of step a') with the solution of step b), and mixing the obtained solution for a period of time sufficient to allow the labelled preparation to adsorb into the air-liquid interface and form a surface film,
d) detecting the signal produced by the labelled preparation adsorbed into interfacial air-liquid interface, and
e) comparing the signal obtained in step d) with the signal produced either by the standard surfactant, or by the surfactant preparation in the absence of activator.

Non-limiting examples of surfactant inhibitors are proteins, such as bovine serum albumin (BSA), fibrinogen, fibrin, C-reactive protein, inmunoglobulines, plasmatic lipoproteins and hemoglobin; lysophospholipids, free fatty acids, cholesterol, bile salts, and complexes, such as serum, plasma, meconium, tobacco residues, asbestos, silica particles, etc. These substances have been studied as inhibitor agents and have been related to several pathologies.

Moreover, the method of the invention also allows the assessment of a surfactant activator, either in the presence or in the absence of a surfactant inhibitor. In the case that the influence of the activator is to be tested in the absence of an inhibitor, the method is carried out in an analogous approach to the one described above, by replacing the surfactant inhibitor by the activator. On the other hand, when the evaluation of the surfactant activator is to be carried out in the presence of a surfactant inhibitor, this method can be carried out preferably following two similar approaches.

Thus, in a first approach, the method comprises the following steps:
a") incubating the test preparation with a detectable substance to obtain a labelled preparation which provides a detectable signal, and adding the activator,
b') preparing an aqueous solution comprising a non-surface active extinguishing agent which is able to block the detectable signal of the substance in step a"), and adding the inhibitor,
c) combining the labelled preparation of step a") with the solution of step b'), and mixing the obtained solution for a period of time sufficient to allow the labelled preparation to adsorb into the air-liquid interface and form a surface film,
d) detecting the signal produced by the labelled preparation adsorbed into interfacial air-liquid interface, and
e) comparing the signal obtained in step d) with the signal produced either by the standard surfactant, or by the surfactant preparation in the absence of activator.

Alternatively, the method comprises the following steps:
a') incubating the test preparation with a detectable substance to obtain a labelled preparation which provides a detectable signal, and adding the inhibitor,
b") preparing an aqueous solution comprising a non-surface active extinguishing agent which is able to block the detectable signal of the substance in step a'), and adding the activator,
c) combining the substances of step a") with the solution of step b"), and mixing the obtained solution for a period of time sufficient to allow the labelled preparation to adsorb into the air-liquid interface and form a surface film,
d) detecting the signal produced by the labelled preparation adsorbed into interfacial air-liquid interface, and
e) comparing the signal obtained in step d) with the signal produced either by the standard surfactant, or by the surfactant preparation in the absence of activator.

In a preferred embodiment the activator is incubated with the test preparation and the detectable substance.

Non-limiting examples of surfactant activators are polyethylene glycol, hyaluronic acid, dextran, chitosan, and hyaluronic acid.

### EXAMPLES

The following examples are provided for illustrative means, and are not meant to be limiting of the present invention.

### A) Surface activity of surfactant preparations

Bulk solution: All the assays were performed in buffered solutions at physiologic ionic strength (NaCl 150 mM). Unless otherwise indicated, the experiments were made in a solution containing NaCl 140 mM, KCI 5 mM, MgCl₂ 1 mM, CaCl₂ 2 mM, HEPES 10 mM at pH 7.

### 1.1) DPPC and mixtures thereof

Multilamellar suspensions of natural isolated phospholipids, or synthetic phospholipids such as 1 ,2-dipalmitoyl-sn-glycero-3-phosphocholine (DPPC), 1-palmitoyl-2-oleoyl-sn-glycero-3-[phospho-rac-(1-glycerol) (POPG) and their mixtures, were prepared from a chloroform/methanol 3:1 (v/v) stock solution (10 mg/ml). Required amounts of the lipids were mixed and dried under a N₂ flow and afterwards, 2 hours under vacuum to remove the organic solvent. Dry lipid films are then hydrated by addition of the required volume of bulk buffered solution to obtain the final lipid concentration (typically 5 mg/ml). Multilamellar liposomes are obtained by swelling of the lipids during 1 h at a temperature above the phase transition temperature of the corresponding lipid and periodical mechanical shaking. For suspensions containing DPPC a temperature of 45 °C is used, which is above its phase transition temperature (41 °C). Concentration of the resulting lipid suspensions was estimated by phosphorus determination or choline enzymatic determination as described in G. Rouser, et al. "Quantitative analysis of phospholipids by thin-layer chromatography and phosphorus analysis of spots." Lipids 1996, vol. 1, pp. 85-6 and M.N. Nanjee, et al. "Enzymatic fluorometric procedure for phospholipid quantification with an automated microtiter plate fluorometer." Clin Chem 1991, vol. 37, pp. 868-74.

### 1.2) Native surfactant

Native surfactant was obtained by tracheal lavaging of slaughtered adult pigs with ice-cold 0.9% NaCl solution. Surfactant was isolated from alveolar lavage by centrifugation at 1000 x g for 5 min to remove debris and then the supernatant was sedimented at 105000 x g for 1 h. The obtained pellet was then reconstituted and centrifuged again in a discontinuous NaBr density gradient at 120000 x g during 1 h. (H.W. Taeusch, et al. "Inactivation of pulmonary surfactant due to serum-inhibited adsorption and reversal by hydrophilic polymers: experimental." Biophys J 2005, vol. 89, pp.1769-79). Concentration of purified surfactant was estimated by phosphorus determination, or enzymatic determination of choline (see section 1.1).

### 1.3) Lamellar Body-like Particles

Lamellar Body-like Particles (LBPs) were collected from isolated and purified rat alveolar type II (AT II) cells cultured on 10 cm ∅ Petri dishes, which were stimulated with 100 µM ATP and 10 nM phorbol 12-myristate 13-acetate. With this kind of stimulation AT II cells release a considerable amount of their surfactant phospholipids together with the surfactant proteins A, B and C. For details on rat AT II cells isolation see L.G. Dobbs, et al. "An improved method for isolating type II cells in high yield and purity." Am Rev Respir Dis 1986, vol. 134, pp. 141-5. Disruption of the LBPs native structure to obtain inactivated LBPs has been performed by strong shaking of a 3 cm Petri dish containing 1 ml of the LBPs solution for 2 h at 37 °C. Concentration of LBPs suspensions was estimated by phosphorus determination, or enzymatic determination of choline (see section 1.1).

### 2) Surfactant labelling with Bodipy-PC

To label lipid suspensions, including purified surfactant, 1,2-bis-(4,4-difluoro-5,7-dimethyl-4-bora-3a,4a-diaza-s-indacene-3-undecanoyl)-sn-glycero-3-phosphocholine (Bodipy-PC) was dissolved in DMSO to yield a concentration of 1 mg/ml. Surfactant materials as obtained above were adjusted to 0,5 mg/ml, except LBPs which were used at the concentration they were purified (typically 0,015 mg/ml) to preserve their structural integrity as much as possible avoiding further centrifugations. All surfactants were stained by incubation with Bodipy-PC at 37 °C for 2 h to obtain a final molar ratio of 1 or 4 % (dye/surfactant). After staining, non incorporated dye can be removed by centrifugation at 120000 x g. Pellet was diluted with the bulk solution to the final working concentration. This treatment is not recommended for LBPs to avoid disruption of their native structure.

Alternatively, dye was added to the organic solution of DPPC and other lipids mixtures. Bodipy-PC was dissolved in a chloroform/methanol (3:1 v/v) solution to a concentration of 1 mg/ml. A volume of this preparation was added to the chloroformic lipid solution to yield a molar ratio of 1 or 4 % (dye/lipid). The samples were then dried and rehydrated as indicated in section 1.1.

Solutions containing fluorescent dye must be protected from light along the labelling process.

### 3) Preparation of the bulk solution containing Brilliant Black®

The light extinguisher Brilliant Black® (BB) was prepared in a buffered solution containing NaCl 140mM, KCI 5mM, MgCl₂ 1mM, CaCl₂ 2mM, HEPES 10mM, pH 7 (bulk solution), to obtain a final concentration of 5 mg/ml. For experiments in plate, an initial bulk solution was prepared at 6.25 mg/ml of BB. By mixing 80 µl of this bulk solution with 20 µl of the surfactant suspension, without BB, a final 5 mg/ml concentration of BB was reached.

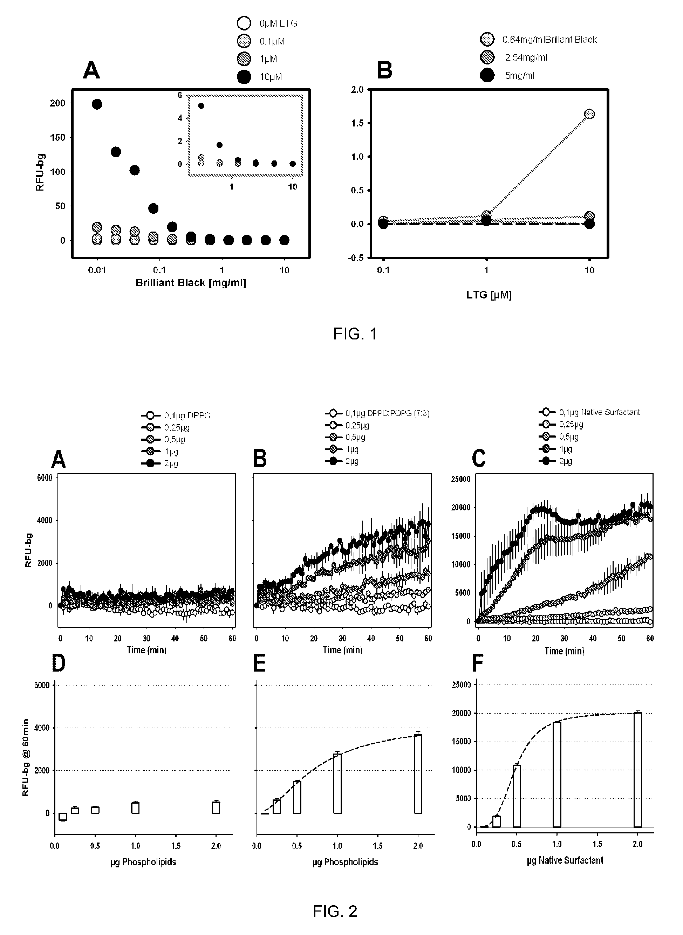

Concentration of the extinguisher was determined by checking the molar extinction coefficient of the extinguisher at the emission and excitation wavelengths used to monitor the fluorescence from labelled surfactants (Bodipy-PC, λₑₓ= 485 nm, λₑₘ=540 nm). To be sure the selected concentration of the dye was enough to block all the fluorescence coming from the subphase, the fluorescence from the hydrophilic dye LysoTracker Green DND-26 (LTG, λₑₓ= 504 nm, λₑₘ=511 nm) was measured in the presence of BB. Fluorescence from solutions of LTG (0.1, 1 and 10 µM) was recorded in the presence of different concentrations of BB (from 0.01 to 10 mg/ml). FIG.1 shows the ability of Brilliant Black to extinguish fluorescence from the water soluble dye LysoTracker Green DND-26 (LTG). Inset in A (enlarged scale) and B (replot of same data as in A) show that 5 mg/ml BB is sufficient to fully extinguish bulk fluorescence of LTG even at the highest concentrations of LTG tested.

### 4) Measurement of the surfactant activity

Measurements of the surfactant activity were performed in a plate reader, either a TECAN GENios Plus from TECAN GmbH (Switzerland) or a FLUOSTAR Optima bmg, using 96-well microtiter plates made of transparent polystyrene (A. Hartenstein GmbH, Austria). These instruments allow control of the temperature (37 °C) and an orbital shaking of the entire plate in between single measurement during a kinetic cycle.

Wells were filled with 80 µl of the aqueous solution containing 6.25 mg/ml of BB as described above. The plate was inserted in the microplate reader and, after reaching 37 °C, the obtained signals were taken as background values. Measurements were performed from the top of the wells ("top reading" mode) using appropriated filter set (band-pass excitation and emission filters, 485 ± 10 and 540 ± 10 nm, for Bodipy-PC).
Afterwards, the plate was taken out of the instrument and 20 µl of solution containing the fluorescently labelled lipid (0.1, 0.25, 0.5, 1 and 2 µg of phospholipid) was injected into the bulk solution of 3 wells (yielding a final concentration of BB of 5 mg/ml). Thereafter, a kinetic cycle of fluorescence measurements was started (cycle time =1 min, orbital shaking = 30 s). Immediately afterwards, the same procedure was repeated with the only difference of 3 sec (instead of 30) of orbital shaking. Values of fluorescence were corrected for background and plotted as the mean ± standard error of 3 repl icates.

### 4.2) Native surfactant (NS)

The same method as described in section 4.1 was used to measure surface activity of native surfactant. Amounts of 0.1 to 2 µg of surfactant in 20 µl of the bulk solution were injected into 80 ml of the bulk solution containing the BB (6.25 mg/ml) after reading the background of the BB. As previously, experiments were performed in triplicates.

### 4.3) Lamellar Body-like Particles (LBPs)

To assay the surface activity of LBPs and disrupted ("treated") LBPs (see section 1.3) the same procedure as in 4.1 was used. 0.5µg LBPs in 20 µl of the bulk solution were applied into the wells containing the solution of BB (6.25 mg/ml) after reading the background signal. Experiments were performed in triplicates.

FIG. 2 shows the surface adsorption kinetics of the surfactants as described above: pure DPPC (FIG. 2 A); DPPC:POPG (7/3, w/w) lipid mixture reconstituted as multilamellar suspensions (FIG. 2 B), and NS (FIG. 2 C, note the different scaling in C compared to A and B). DPPC has extremely slow adsorption kinetics, if at all. Suspensions made of DPPC:POPG can adsorb into the air-liquid interface and form a surface film. Finally, NS shows dramatic and rapid adsorption into the interfacial surface. In FIG. 2 D, 2 E and 2 F, fluorescence values at 60 min (data from experiments FIG. 2 A to 2 C) have been plotted as a function of surfactant phospholipid concentration. Adsorbing surfactants (i.e. DPPC:POPG and NS) showed time- and dose-dependency.

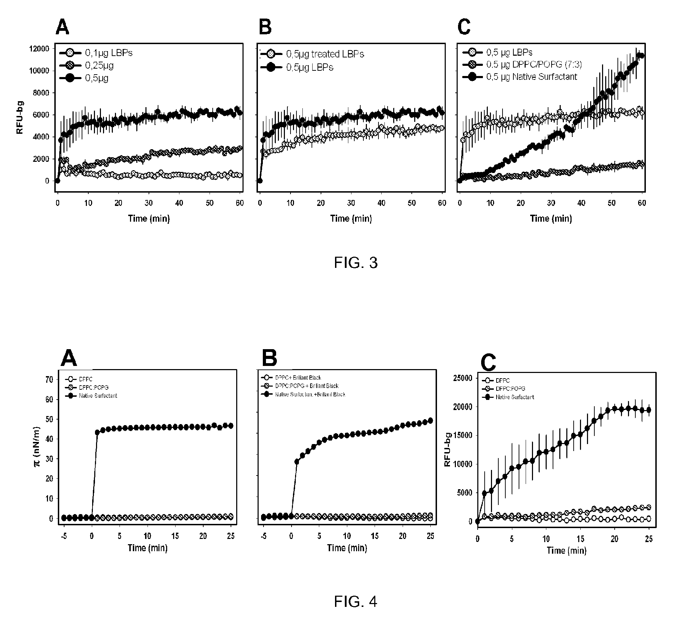

FIG. 3 shows surface adsorption kinetics of Lamellar Body-like Particles (LBPs) collected from stimulated AT II cells as detected in the fluorescent plate reader assay. FIG. 3 A shows kinetics of adsorption at different amounts of LBPs. FIG. 3 B shows LBPs (black) and interface-treated LBPs (grey - see text) adsorption kinetics are very similar. Intact LBPs adsorbed better than treated LBPs, although a loss of LBPs during treatment cannot be excluded. In FIG. 3 C adsorption kinetics of LBPs (data form A) is compared with the kinetics of DPPC:POPG lipid suspension and NS at equal amounts (data from Fig. 2 B and C). Freshly secreted surfactant (LBPs) revealed a faster adsorption than NS, but they quickly reached a plateau which is not seen with NS despite same concentrations used.

### B) Surface activity of surfactant preparations by comparative Wilhelmy Balance measurements

Interfacial adsorption of surfactant samples was followed on a Teflon-made Wilhelmy balance, thermostatized at a temperature of 25 or 37°C. The diameter of the trough was 2 cm and the total volume of subphase was 1.5 ml. The experiments were started by injection of a small volume (typically 5-50 µl) of a given surfactant preparation into the subphase, through an oblique lateral perforation. Continuous stirring with a 2 mm long magnetic bar ensures the rapid mixing of the applied sample in the bulk subphase. Surface pressure-time kinetics were followed through continuous acquisition of surface pressure data with a Wilhelmy plate made of Whatman #1 paper connected to a pressure sensor. Experiments were made injecting 10 µl of a 15 mg/ml suspension of surfactant or lipids into a bulk solution of NaCl 140mM, KCI 5mM, MgCl₂ 1 mM, CaCl₂ 2mM, HEPES 10mM, pH 7 in the absence or in the presence of BB, at 37°C.

FIG. 4 A shows Wilhelmy Balance evaluation of surface pressure increase due to surfactant adsorption (surfactant injection at time 0); FIG. 4 B Wilhelmy balance evaluation of surface pressure increase due to surfactant adsorption in presence of BB into the subphase; the adsorption kinetics as followed in the balance changed slightly, but not the essential behavior of all 3 materials studied nor their endpoints reached. FIG. 4 C is a replot of data from Fig. 2 A, 2 B and 2 C (2 µg) for a direct comparison between fluorescence and surface pressure measurements under comparable conditions.

### C) Influence of experimental parameters on the surface activity of surfactant preparations

The influence of pH, ionic strength and calcium was tested on the surface activity of native surfactant (NS). Native surfactant was purified and labelled as previously described with the only difference of the final molar ratio, which in this case was 1% dye/surfactant. All experiments concerning the effect of ionic strength, calcium, pH were performed injecting 3µg of surfactant phospholipids per well. All experiments were carried out in triplicates, in the same way as described before, filling the wells with 80µl of the corresponding bulk solution described below for each experiment containing 6.25mg/ml of BB. The plate was inserted in the microplate reader after reaching 37°C and the background was measured. Immediately after, 20µl of the corresponding bulk solution containing the labelled surfactant was injected.

### C.1) Ionic strength

Bulk solution: 10mM Hepes buffer solution, pH 7, was prepared containing different concentrations of NaCl: 0mM; 50mM; 100mM; 150mM; 250mM; 500mM and 1M

### C.2) Calcium

Bulk solution: 10mM Hepes, 150mM NaCl buffer solution, pH 7, was prepared containing different concentrations of CaCl₂: 0mM; 0.2mM; 0.5mM; 150mM; 1 mM; 2mM and 5mM. To achieve a buffer solution 0mM Ca²⁺, a calcium chelator was added, EDTA 2mM.

### C.3) pH

Bulk solution: 150mM NaCl; 5mM Tris; 5mM Mes; 5mM Sodium Acetate. pH was adjusted to reach the following units: 3; 4; 5; 6; 7; 8; 9; 10.
Two different experiments were performed:
1) Surfactant was diluted in the bulk solution at each pH, and then injected into the bulk solution containing BB with the same pH.
2) Surfactant was diluted in 10µl bulk solution pH 7, and then injected into 90µl of the different pH bulk solutions containing BB. In this case, pH was measured in each well to verify the final pH.

For each experimental parameter, a kinetic cycle of fluorescence measurements was performed with the following conditions set in a FLUOSTAR OPTIMA bmg microplate reader:
Temperature: 37°C
Orbital shaking: 3"; 200rpm; shaking width: 3; shaking before each cycle
N° of cycles: 20-35
N° of flashes: 3
Positional delay: 0.2
Measurement start time: 3
Gain: 1200-1500

The results obtained were the fluorescence values corrected for background and plotted as the mean of 3 replicates.

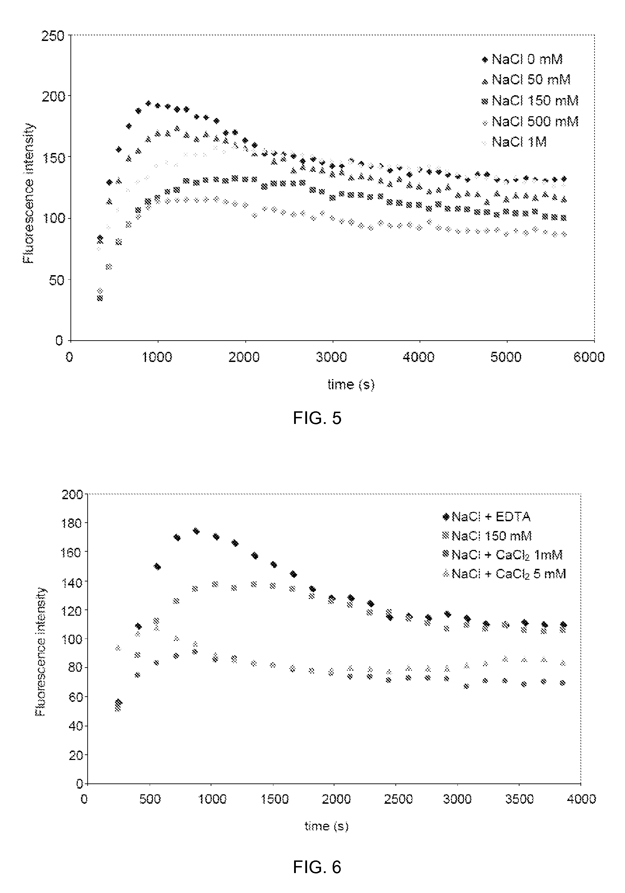

FIG. 5 shows the influence of ionic strength on the surface activity of NS. At low ionic strength surfactant promotes a rapid adsorption of material at the interface, followed by a partial desorption of excess material towards the subphase. With increasing ionic strength there is a reduction of the total material adsorbed at the initial step, so that there is practically no adsorption of excess material. Maximal adsorption with no desorption occurs at physiological ionic strength, NaCl 150 mM. The results indicate that in the absence of salt, probably some weak ionic interactions make that an excess of material is temporarily associated with the interface and later removed upon rearrangement of the surface film. This is prevented under physiological ionic conditions.

FIG. 6 shows the influence of calcium on the surface activity of NS. Addition of millimolar concentrations of calcium to the bulk solution decreases the amount of surfactant associated with the interface. Addition of EDTA, a calcium chelator, increases the amount of surfactant adsorbing to the interface, indicating that there are basal effects due to micromolar or submicromolar concentrations of calcium already present in native surfactant.

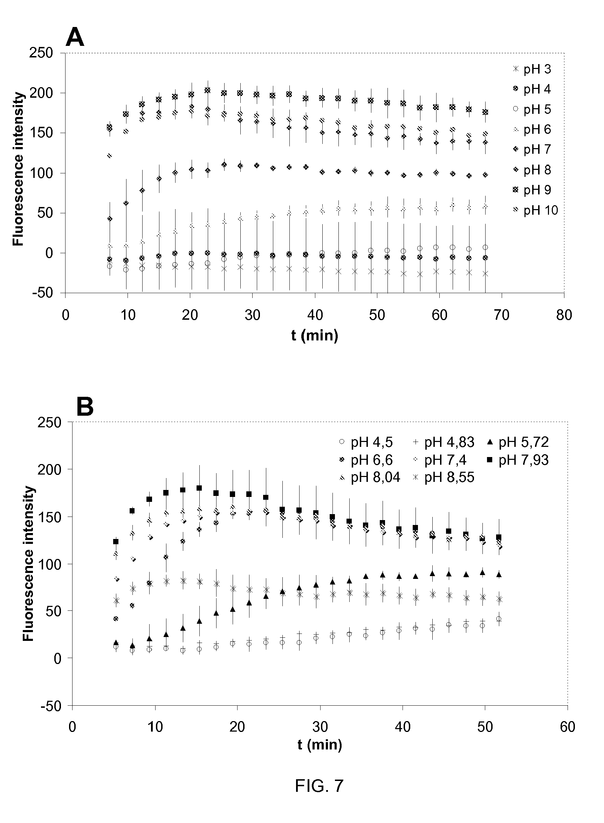

FIG. 7 shows the influence of pH on the surface activity of NS. Acidic pH strongly inhibits formation of surfactant films when surfactant is preincubated at different pH's and then introduced into subphases bearing the same pH. Maximal activity is achieved maintaining surfactant and the subphase in the pH range 7-10 (FIG. 7 A). A similar effect is observed if surfactant diluted at pH 7 is assessed for surface adsorption by injection into subphases adjusted to different pH (FIG. 7 B).

### D) Surface activity of surfactant preparations in the presence of an inhibitor

To test the effect of serum proteins and bovine serum albumin (BSA) in the activity of various surfactants experiments as described in 4.1 were repeated in the presence of different concentrations of serum proteins or different concentrations of albumin. All experiments were carried out in triplicates, in the same way as described before, filling the wells with 80µl of the corresponding bulk solution described below for each experiment containing 6.25mg/ml of BB. The plate was inserted in the microplate reader after reaching 37°C and the background was measured. Immediately after, 20µl of the corresponding bulk solution containing the labelled surfactant was injected.

### D.1) Serum proteins

Surfactants: Native surfactant (NS), clinical surfactant Curosurf®, which was labelled following the same protocol as for native surfactant.
Bulk solution: 10mM Hepes buffer solution, 150 mM NaCl, pH 7 was prepared containing different concentrations of serum proteins: 0mg/ml; 15.5 mg/ml; 31 mg/ml final protein concentration. The concentration of total protein in original serum obtained from porcine blood was determined by the Lowry method.

### D.2) Albumin (BSA)

Surfactants: Native surfactant (NS). Clinical surfactant Curosurf® was labelled following the same protocol as for native surfactant.
Bulk solution: 10mM Hepes buffer solution, 150 mM NaCl, pH 7 was prepared containing different concentrations of BSA: 0 mg/ml; 0.5 mg/ml; 1 mg/ml; 1.5 mg/ml final protein concentration.

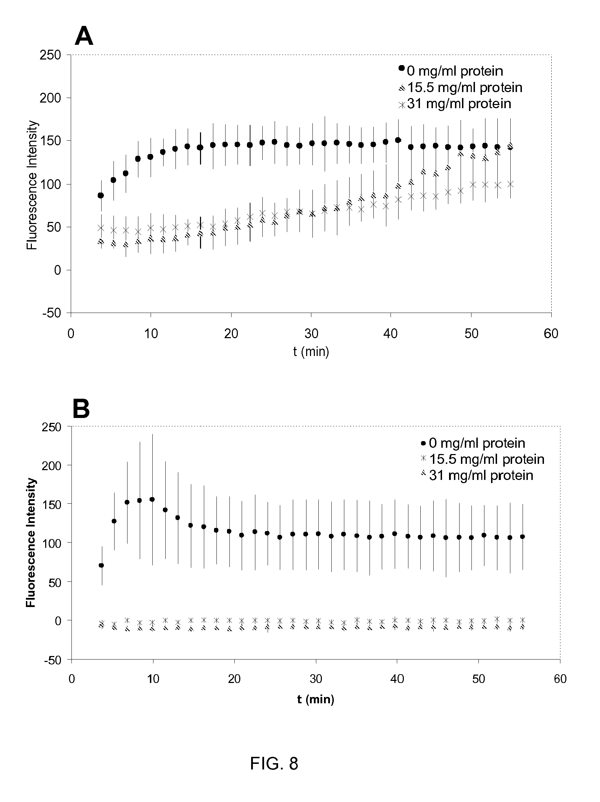

FIG. 8 shows the influence of the serum proteins on the surface activity of NS (FIG.8 A) and of Curosurf® (FIG. 8 B). Presence of serum inhibits adsorption of natural whole surfactant, particularly at the initial steps. As a comparison, similar serum concentrations totally deactivate Curosurf®, one of the most used clinical surfactant preparation available today for the treatment of surfactant-based respiratory pathologies.

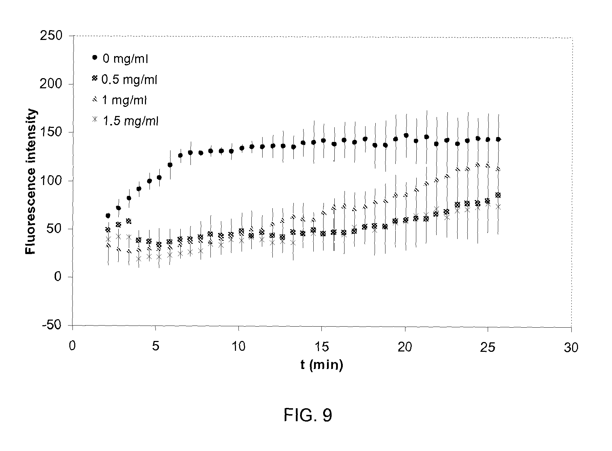

FIG.9 shows the influence of the surfactant inhibitor BSA on the surface activity of NS.

## Claims

1. A method for evaluating the surface activity of a test preparation comprising the following steps:
a) incubating the test preparation with a detectable substance to obtain a labelled preparation which provides a detectable signal,
b) preparing an aqueous solution comprising a non-surface active extinguishing agent which is able to block the detectable signal of the substance in step a),
c) combining the labelled preparation of step a) with the solution of step b), and mixing the obtained solution for a period of time sufficient to allow the labelled preparation to adsorb into the air-liquid interface and form a surface film,
d) detecting the signal produced by the labelled preparation adsorbed into interfacial air-liquid interface, and
e) comparing the signal obtained in step d) with the signal produced by a standard surfactant.

2. The method according to claim 1, wherein the test preparation is a pulmonary surfactant.

3. The method according to claim 2, wherein the aqueous solution is a buffered solution adjusted to meet physiological conditions.

4. The method according to any of the claims 1-3, wherein the detectable substance is a molecule being or containing a detectable moiety, and this molecule interacts stably with the test preparation.

5. The method according to any of claims 1-4, wherein the detectable substance comprises a fluorophore, and the extinguishing agent is a fluorescence extinguisher.

6. The method according to claim 5, wherein the detectable substance comprising a fluorophore is 1,2-bis-(4,4-difluoro-5,7-dimethyl-4-bora-3a,4a-diaza-s-indacene-3-undecanoyl)-sn-glycero-3-phosphocholine, and the fluorescence extinguisher is tetrasodium (6Z)-4-acetamido-5-oxo-6-[[7-sulfonato-4-(4-sulfonatophenyl)diazenylnaphthalen-1-yl]hydrazinylidene]-naphthalene-1,7-disulfonate.

7. Kit for evaluating the surface activity of a test preparation by the method as defined in any of the claims 1-6 comprising:
a) a detectable substance with molecular properties making it suitable for stable incorporation into test preparations,
b) a solution comprising a non-surface active extinguishing agent which is able to block the detectable signal of the substance in step a), and
c) a standard surfactant.

8. A method for evaluating the surface activity of a test preparation comprising carrying out the method according to any of claims 1-6 and at least a method for measuring the surface tension.

9. A method for determining the influence of an experimental parameter on the surface activity of a surfactant preparation, comprising evaluating the surface activity according to the method as defined in any of the claims 1-6 in response to variations of the experimental parameter.

10. The method according to claim 9, wherein
when the experimental parameter is ionic strength, then the method further comprises the step of adding a ionic salt at different concentrations to the aqueous solution of step b);
when the experimental parameter is pH, then the method further comprises the step of adding different buffers to the aqueous solution of step b); and
when the experimental parameter is presence of Ca, then the method further comprises the step of adding a calcium salt at different concentrations to the aqueous solution of step b).

11. A method for determining the influence of a surfactant inhibitor on the surface activity of a surfactant preparation, the method comprising evaluating the surface activity according to the method as defined in any of the claims 1-6, further comprising the step of adding the inhibitor either to the labelled preparation of step a), or alternatively, to the aqueous solution of step b), and in step e) comparing the signal obtained in step d) with the signal produced either by the standard surfactant, or by the surfactant preparation in the absence of inhibitor.

12. The method according to claim 9, wherein the inhibitor is bovine serum albumin (BSA).

13. A method for determining the influence of a surfactant activator on the surface activity of a surfactant preparation, the method comprising evaluating the surface activity according to the method as defined in any of the claims 1-6, further comprising the step of adding the activator either to the labelled preparation of step a), or alternatively, to the aqueous solution of step b), either in the presence or absence of an inhibitor, and in step e) comparing the signal obtained in step d) with the signal produced either by the standard surfactant, or by the surfactant preparation in the absence of activator.

14. A method for screening a compound library to determine the ability of the compounds to inhibit or activate the surface activity of a surfactant preparation, the method comprising evaluating the surface activity according to the method as defined in any of the claims 1-6, further comprising the step of adding each compound to be screened either to the labelled preparation of step a), or alternatively, to the aqueous solution of step b).

15. A method for controlling the quality of a surfactant preparation, the method comprising evaluating the surface activity according to the method as defined in any of the claims 1-6.
